Europäisches Patentamt

European Patent Office

Office européen des brevets

(19)

(11) Veröffentlichungsnummer : **0 082 431**
**B1**

(12) # EUROPÄISCHE PATENTSCHRIFT

(45) Veröffentlichungstag der Patentschrift :
**05.03.86**

(51) Int. Cl.⁴ : **A 61 N 1/38, A 61 B 5/04**

(21) Anmeldenummer : **82111426.1**

(22) Anmeldetag : **09.12.82**

(54) Einrichtung zur Anzeige des Stromverlaufs eines Defibrillatorimpulses.

(30) Priorität : **21.12.81 DE 3150534**

(43) Veröffentlichungstag der Anmeldung :
**29.06.83 Patentblatt 83/26**

(45) Bekanntmachung des Hinweises auf die Patenterteilung : **05.03.86 Patentblatt 86/10**

(84) Benannte Vertragsstaaten :
**BE CH DE FR GB IT LI NL**

(56) Entgegenhaltungen :
**EP-A- 0 038 080**
**DE-A- 2 651 031**
**DE-A- 2 713 390**
**DE-A- 2 827 729**
**DE-A- 3 035 732**
**GB-A- 1 535 829**
**US-A- 3 862 636**

(73) Patentinhaber : **HONEYWELL MEDICAL ELECTRONICS B.V.**
**I.B.C.-Weg 1**
**NL-5683 PK Best (NL)**

(72) Erfinder : **Lambert, Willibrordus J.S.**
**Johannes de Kortstraat 43**
**Eersel (NL)**

(74) Vertreter : **Rentzsch, Heinz et al**
**Honeywell Europe S.A. Holding KG Patent- und Lizenzabteilung Kaiserleistrasse 55**
**D-6050 Offenbach am Main (DE)**

EP 0 082 431 B1

## Beschreibung

Beim Defibrillieren eines Patienten wird üblicherweise sein EKG vor und nach der Behandlung aufgezeichnet. Außerdem mißt man die von Hand einstellbaren Defibrillatorenergie. Zur besseren Überwachung und Beurteilung einer Defibrillation wäre die Aufzeichnung des zeitlichen Stromverlaufs des Defibrillationsimpulses nützlich. Hieraus könnte man den Spitzenwert des Stroms, den elektrischen Widerstand des Patienten, sowie die ihm zugeführte Energie bestimmen. Aufgabe der Erfindung ist es, den zeitlichen Verlauf des Defibrillatorimpulses unmittelbar während oder im Anschluss an die Defibrillation sichtbar anzuzeigen.

Diese Aufgabe wird gelöst durch die im Anspruch 1 gekennzeichnete Erfindung. Da der Defibrillatorimpuls von beispielsweise 6 ms sehr kurz ist im Vergleich zu einzelnen Bestandteilen (Zacken) des EKG würde er bei ungedehnter Aufzeichnung nur als Strich innerhalb des EKG erscheinen und folglich seinen zeitlichen Verlauf nicht erkennen lassen. Die Erfindung sieht deshalb eine Zeitdehnung des Defibrillatorimpulses vor und blendetihn derart in die fortlaufende Aufzeichnung des EKG ein, daß das EKG sowohl vor als nach der Defibrillation ungestört aufgezeichnet wird. Bei Registrierung des EKG mit einer Papiergeschwindigkeit von 2,5 cm/s empfiehlt es sich den Defibrillatorimpuls von 6 ms um den Faktor 100 zu dehnen und für eine Zeitspanne von etwa 1s in die EKG-Aufzeichnung einzublenden. Weitere vorteilhafte Ausgestaltungen der Erfindung ergeben sich aus den Unteransprüchen.

Die Erfindung wird nachfolgend anhand eines in der Zeichnung wiedergegebenen Ausführungsbeispiels erläutert. Dabei zeigt

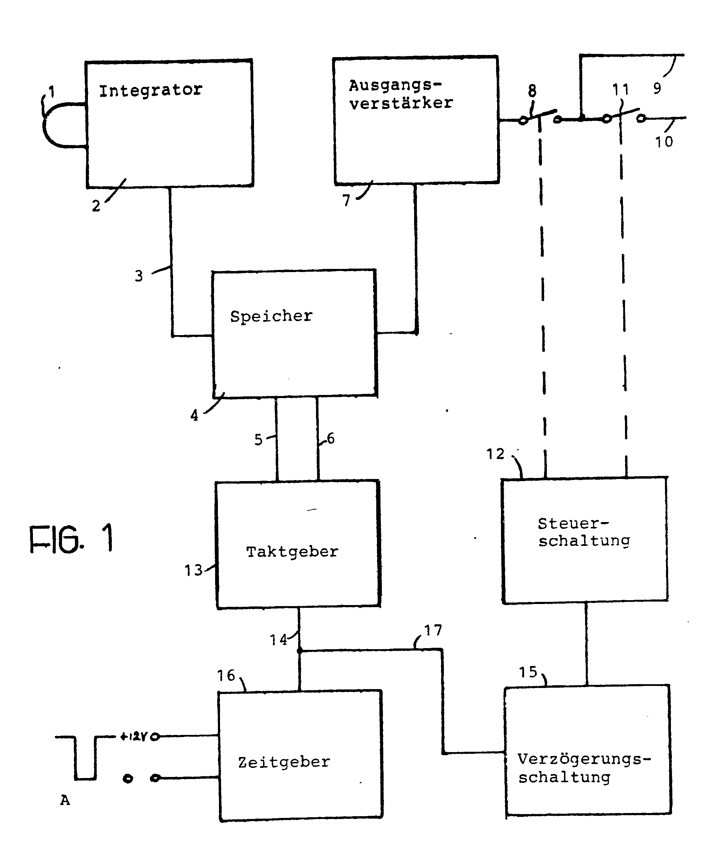

Figur 1 die wesentlichen Schaltungsteile als Zusatz zu einem üblichen EKG-Schreiber oder Monitor und

Figur 2 den zeitlichen Signalverlauf an verschiedenen Punkten der Schaltungsanordnung sowie die Gesamtaufzeichnung des EKG-Signals einschließlich Defibrillatorimpuls.

Der Defibrillator-Stromstoß für den Patienten wird üblicherweise von einem Übertragungskondensator geliefert, der zuvor auf eine entsprechende Spannung aufgeladen wurde und dann beidpolig an den Patientenstromkreis gelegt wird. In diesem Stromkreis liegt zumeist eine Drosselspule. Zur Ableitung der dem Verlauf des Defibrillatorstroms entsprechenden elektrischen Größe kann ein Stromwandler in den Patientenstromkreis eingeschaltet werden. Einfacher ist es jedoch, die den Stromverlauf durch den Patienten mitbestimmende Drosselspule mit einer zusätzlichen Auskoppelwicklung 1 zu versehen und dieser einen Integrator 2 nachzuschalten. Durch Integration der mittels der Wicklung 1 ausgekoppelten Spannung erhält man ein dem zeitlichen Verlauf des Patientenstroms entsprechendes Signal am Ausgang 3 des Integrators 2. Dieses Signal gelangt zu einem Analogspeicher 4, dessen beiden Takteingängen 5 und 6 unterschiedliche Taktfrequenzen zugeführt werden können. Über einen Ausgangsverstärker 7 und eine Schaltstrecke 8 kann der Analogspeicher mit dem Schreibeingang 9 eines EKG-Schreibers oder Monitors verbunden werden. Das EKG-Signal des Patienten kommt an einer Eingangsklemme 10 an, die über eine zweite Schaltstrecke 11 ebenfalls mit dem Schreibeingang 9 des Anzeigegerätes in Verbindung steht.

Die beiden Schaltstrecken 8 und 11 werden wechselseitig durch eine Steuerschaltung 12 umgeschaltet. Sie sind in der Zeichnung schematisch als Schaltkontakte dargestellt, werden jedoch in der Praxis vorzugsweise elektronische Schaltstrecken beispielsweise Feldeffekttransistoren sein. Ein Taktgeber 13 liefert auf den beiden Taktleitungen 5 und 6 Taktfolgen CL unter schiedlicher Frequenz, wobei eine hochfrequente Taktfolge von beispielsweise 25 kHz zum Einschreiben des Integratorausgangssignals in den Analogspeicher 4 dient, während das Auslesen des Speicherinhalts und dessen Übertragung in den Verstärker 7 mit einer niederfrequenten Taktfolge von beispielsweise 250 Hz erfolgt. Zwischen den Steuereingang 14 des Taktgebers 13 und den Steuereingang der Steuerschaltung 12 ist eine Verzögerungsschaltung 15 eingeschaltet. Ein Zeitgeber 16 ist dem Steuereingang 14 und der Verzögerungsschaltung 15 vorgeschaltet.

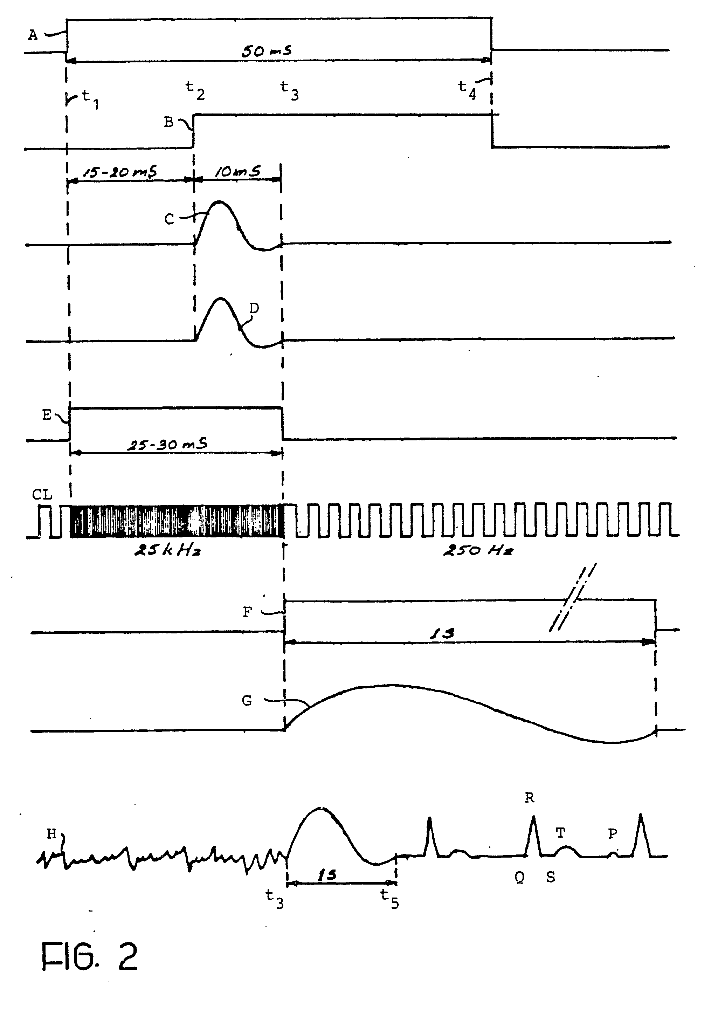

Die Arbeitsweise dieser Schaltung soll nachfolgend anhand von Figur 2 beschrieben werden. Dabei wird angenommen, daß zwischen einem die Defibrillation auslösenden Steuerimpuls und dem Beginn des Stromflusses über den Patienten eine Zeitspanne verstreicht, deren Dauer von der Speisespannung des Defibrillatorrelais abhängig ist. Diese Zeitspanne wird durch den Zeitgeber 16 kompensiert.

Es sei angenommen, daß zur Zeit $t_1$ das Kommando zum Defibrillieren in Form eines Impulses A ausgelöst wird. Dieser Impuls dauert im Beispiel 50 ms. Die Umschaltung des den Patientenstrom liefernden Übertragungskondensators mittels eines Relais erfolgt nach einer Ansprechverzögerung von 15 bis 20 ms zum Zeitpunkt $t_2$. Das Defibrillatorrelais bleibt entsprechend dem Signal B bis zum Ende des Steuerimpulses A zum Zeitpunkt $t_4$ angesprochen. Mit der Umschaltung des Übertragungskondensators beginnt im Zeitpunkt $t_2$ der Patientenstrom C zu fließen. Der Stromfluss dauert etwa 6 bis 10 ms und ist zum Zeitpunkt $t_3$ abgeklungen. Über die Koppelspule 1 wird eine der Spannung an der Drosselspule entsprechende Spannung ausgekoppelt und liefert am Ausgang 3 des Integrators 2 das Signal D. Dieses Signal wird mit einer hohen Taktfrequenz von beispielsweise 25 kHz abgetastet und in den Analogspeicher 4 eingegeben. Der Auslöseimpuls A stößt hierzu einen spannungsabhängigen Zeitgeber 16 an, der zum Zeitpunkt $t_1$ an seinem Ausgang dem Taktgeber 13 ein Steuersignal zuführt, welches den Taktgeber auf die hohe Abtastfrequenz von beispielsweise 25 kHz umschaltet. Nach Ablauf der Ansprechverzögerung von 15 bis 20 ms zuzüglich der maximalen Patientenstromdauer von 10 ms d. h. insgesamt nach 25 bis 30 ms gibt der Zeitgeber 16 ein zweites Signal an den Steuereingang des Taktgeber 13 und schaltet diesen zum Zeitpunkt $t_3$ wieder auf die niedrigere Taktfrequenz von beispielsweise 250 Hz um. Das Ausgangssignal des spannungsabhängigen Zeitgebers 16 ist mit E bezeichnet. Sofern die Ansprechverzögerung des Defibrillatorrelais von der Höhe seiner Speisespannung unabhängig ist, kann der Zeitgeber 16 mit einer vorgegebenen, also nicht spannungsabhängigen, Verzögerungszeit arbeiten.

Während der Dauer des Ausgangsimpulses E des Zeitgebers 16 wird somit das am Ausgang 3 des Integrators 2 stehende Signal mit hoher Frequenz abgetastet und in den Speicher 4 eingegeben. Die Verzögerung von 25 bis 30 ms stellt sicher, daß auf jeden Fall die gesamte Dauer des Patientenstromstoßes erfaßt und in den Speicher eingeschrieben wird. Am Schluss dieser Einspeicherung soll dieses Signal zeitgedehnt aufgezeichnet und zwar in die EKG-Aufzeichnung eingeblendet werden. Diese erfolgt zuvor beispielsweise mit einer Schreibstreifengeschwindigkeit von 2,5 cm/s in dem das von den EKG-Elektroden gelieferte und verstärkte Signal über die Klemme 10 und die Schaltstrecke 11 dem Schreibeingang 9 des Anzeigegerätes zugeführt wird. Für die Aufzeichnung des Kurvenverlaufs des Defibrillatorimpulses wird zur Zeit $t_3$ die Aufzeichnung des EKG unterbrochen. Hierzu ist an den Ausgang des Zeitbers 16 und damit auch an den Steuereingang des Taktgebers 13 über die Leitung 17 eine Verzögerungsschaltung 15 angeschlossen, die mit dem Ablauf der Verzögerungszeit des Zeitgebers 16, d. h. zum Zeitpunkt $t_3$ angestoßen wird. Die Verzögerungsschaltung 15 hat beispielsweise eine Verzögerungszeit von 1s die in Figur 2 als Impuls F dargestellt ist. Während dieses Impulses F wird von der Steuerschaltung 12 die Schaltstrecke 11 vom EKG-Verstärker unterbrochen und statt dessen die Schaltstrecke 8 zwischen dem Verstärker 7 und dem Schreibeingang 9 des EKG-Schreibers durchgeschaltet. Damit gelangt das Ausgangssignal des Speichers 4 über den Verstärker 7 zum EKG-Schreiber oder Monitor und wird aufgezeichnet. Mit dem Ablauf des Impulses E am Ausgang des Zeitgebers 16 wurde jedoch wie oben erwähnt, zugleich die Taktfrequenz des Taktgebers 13 wieder auf die niedrigere Frequenz von beispielsweise 250 Hz umgeschaltet, so daß der Speicherinhalt mit einer wesentlich niedrigeren, im Beispiel um den Faktor 100 niedrigeren, Frequenz ausgelesen und aufgezeichnet wird. Damit erhält man aus der ursprünglich am Ausgang 3 des Integrators 2 abgenommenen Spannungskurve D eine zeitlich gedehnte Aufzeichnung G. Diese läßt den Stromverlauf des Defibrillatorimpulses deutlich erkennen, so daß dieser hinsichtlich seiner Größe und seiner Kurvenform ausgewertet werden kann. Nach Ablauf der Verzögerungszeit von beispielsweise 1s der Verzögerungsschaltung 15 schaltet die Steuerschaltung 12 die beiden Schaltstrecken 8 und 11 wieder um, so daß nunmehr erneut das EKG-Signal vom EKG Eingang 10 zum Schreibeingang 9 des Aufzeichnungsgerätes gelangt.

Der Kurvenzug H in der letzten Zeile von Fig. 2 zeigt einen typischen Verlauf einer solchen Aufzeichnung. Bis zum Zeitpunkt $t_3$ wird das gestörte EKG und ab $t_5$ das Elektrokardiogramm P, Q, R, S, T aufgezeichnet. Die Aufzeichnung H wird z. Zt. $t_3$ für eine Sekunde entsprechend der Dauer des Ausgangssignales F der Verzögerungsschaltung 15 unterbrochen und durch die zeitgedehnte Aufzeichnung G des Defibrillatorimpulses ersetzt. Zum Zeitpunkt $t_5$ erfolgt die Umschaltung der Schaltstrecken 8 und 11 in die Ausgangslage, so daß nunmehr wiederum das EKG aufgezeichnet wird.

Im gezeigten Ausführungsbeispiel wird als Zwischenspeicher für das Defibrillatorsignal ein Analogspeicher 4, beispielsweise ein ladungsgekoppelter Speicher verwendet, in den ein Signal von 10 ms Dauer eingegeben wird. Um hierbei jegliche Verfälschung der Impulsform zu vermeiden, muß eine genügende Speicherkapazität von wenigstens 100 Worten mit je 6 Bit vorhanden sein. Bei einer Zeitdehnung um den Faktor 100 dauert das Auslesen des Speichers dann etwa 1s. Erfolgt die Aufzeichnung des EKG nicht auf einem Schreibstreifen, sondern auf einem Bildschirm, so ist diesem zur Aufrechterhaltung der Darstellung üblicherweise ein Speicher zugeordnet. Dieser könnte dann auch zur Speicherung des Defibrillatorimpulses benutzt werden. Dies hat jedoch den Nachteil, daß bei der Speicherung des Defibrillatorimpulses das zuvor dort gespeicherte EKG gelöscht und durch den Defibrillatorimpuls ersetzt wird. Günstiger ist deshalb die Verwendung eines besonderen Zwischenspeichers für den Defibrillatorimpuls.

**Patentansprüche**

1. Einrichtung zur Anzeige des zeitlichen Stromverlaufs eines Defibrillatorimpulses, gekennzeichnet durch

a) eine Auskoppeleinrichtung (1) für eine dem Defibrillationsstrom entsprechende elektrische Größe ;

b) einen Speicher (4) zur Aufnahme eines dieser zeitveränderlichen elektrischen Größe entsprechenden Signals ;

c) eine Schaltungsanordnung (13) zur Zeitdehnung des Signals beim Auslesen aus dem Speicher (4) ;

d) Anschlußmittel (9, 10) für ein Anzeigegerät zur Darstellung des zeitgedehnten Signals und zur Wiedergabe des Elektrokardiogramms eines Patienten ;

e) eine Steuerschaltung (12) welche für die Dauer der Anzeige des zeitgedehnten Signals die Wiedergabe des Elektrokardiogramms des Patienten auf dem Anzeigegerät vorübergehend unterbricht.

2. Einrichtung nach Anspruch 1, wobei als Anzeigegerät ein Aufzeichnungsgerät mit einer Papiergeschwindigkeit von etwa 2,5 cm/s eingesetzt ist, dadurch gekennzeichnet, daß der Zeitdehnungsfaktor etwa 100 beträgt.

3. Einrichtung nach Anspruch 1, dadurch gekennzeichnet, daß zur Anzeige ein Bildschirm vorgesehen und diesem ein Zwischenspeicher zugeordnet ist.

4. Einrichtung nach einem der Ansprüche 1 bis 3, dadurch gekennzeichnet, daß der Speicher (4) als Analogspeicher für wenigstens 100 Worte mit 6 Bit Wortlänge ausgebildet ist.

5. Einrichtung nach einem der Ansprüche 1 bis 4, dadurch gekennzeichnet, daß dem Speicher (4) zur Umschaltung von Schreib- auf Lesegeschwindigkeit ein umschaltbaren Taktgeber (13) zugeordnet ist, dessen Steuereingang (14) über einen auf die Ansprechverzögerung des Defibrillators sowie die maximale Patientenstromdauer abgestimmten Zeitgeber (16) an einen die Defibrillation auslösenden Steuerimpulsgeber angeschlossen ist.

6. Einrichtung nach Anspruch 5 mit spannungsabhängiger Umschaltdauer des den Defibrillatorimpuls einschaltenden Relais, dadurch gekennzeichnet, daß der zwischen dem Steuerimpulsgeber und dem Steuereingang (14) des Taktgebers (13) geschaltete Zeitgeber (16) eine von der Speisespannung des Relais abhängige Verzögerungsdauer aufweist.

7. Einrichtung nach Anspruch 5 oder 6, dadurch gekennzeichnet, daß an den Ausgang des Zeitgebers (16) eine die Dauer der Unterbrechung bestimmende Verzögerungsschaltung (15) angeschlossen ist, deren Ausgangssignal der Steuerschaltung (12) zugeführt ist, welche die Signalzufuhr zum Anzeigegerät zwischen einer das EKG-Signal führenden Leitung (10) und dem Ausgang (7) des Speichers (4) umschaltet.

8. Einrichtung nach einem der Ansprüche 1 bis 7, dadurch gekennzeichnet, daß die Auskoppelvorrichtung einen in den Defibrillator/Patienten-Stromkreis eingeschalteten Stromwandler umfaßt.

9. Einrichtung nach einem der Ansprüche 1 bis 7 mit einer Drosselspule im Defibrillator/Patientenstromkreis, dadurch gekennzeichnet, daß die Auskoppelvorrichtung eine mit der Drosselspule gekoppelte Wicklung (1) sowie einen nachgeschalteten Integrator (2) umfaßt.

## Claims

1. Arrangement for displaying the time dependent current wave form of a defibrillator pulse, characterized by

a) coupling means (1) for producing an electrical quantity corresponding to the defibrillator current ;

b) a memory (4) for storing a signal corresponding to said time variable electrical quantity ;

c) a circuit (13) for achieving a time extension of said signal when reading the signal from said memory ;

d) connecting means (9, 10) for an indicating device for displaying the time extended signal and for displaying the electrocardiogram ECG of a patient ;

e) a control circuit (12) which during the displaying of the time extended signal temporarily interrupts the displaying of the ECG of the patient on the indicating device.

2. Arrangement according to claim 1 whereat a recorder having a paper speed of about 2,5 cm/s is used as indicating device, characterized in that the time extension factor is about 100.

3. Arrangement according to claim 1, characterized in that a monitor is used for indicating and an intermediate memory is associated with said monitor.

4. Arrangement according to one of the claims 1 to 3, characterized in that the memory (4) is an analog memory for at least 100 words with 6 bit word lengths.

5. Arrangement according to one of the claims 1 to 4, characterized in that for switching from writing speed to reading speed a switchable clock (13) is associated with the memory (4) and the control input (14) of said clock is by means of a timer (16) connected to a control pulse generator initiating the defibrillation ; with said timer being matched with the switch-on delay of the defibrillator and the maximum period of current flow to the patient.

6. Arrangement according to claim 5 with a voltage dependent switch-over time of the relay which switches the defibrillator on, characterized in that the timer (16) provided between the control pulse generator and the control input of the clock (13) has a delay period which depends on the supply voltage of the relay.

7. Arrangement according to claim 5 or 6, characterized in that a delay circuit (15) is connected to the output of the timer (16) which delay circuit determines the duration of the interruption, with the output signal of said delay circuit being fed to the control circuit (12) which switches the signal supply to the indicating device between a first line (10) carrying the ECG signal and a second line connected to the output (7) of the memory (4).

8. Arrangement according to one of the claims 1 to 7, characterized in that the coupling means includes a current tranformer connected into the defibrillator/patient circuit.

9. Arrangement according to one of the claims 1 to 7, comprising a reactance coil in the defibrillator/patient circuit, characterized in that the coupling means includes a winding (1) coupled to the reactance coil and further comprises an integrator (2) connected to said winding (1).

## Revendications

1. Dispositif pour l'affichage de la variation dans le temps d'une impulsion de courant d'un défibrillateur, caractérisé par :

a) un dispositif de découplage (1) pour une grandeur électrique correspondant au courant de défibrillation ;

b) une mémoire (4) servant à enregistrer un signal correspondant à cette grandeur électrique variable dans le temps ;

c) un montage (13) servant à dilater dans le temps le signal lors de la lecture à partir de la mémoire (4) ;

d) des moyens de raccordement (9, 10) pour un appareil d'affichage pour la représentation du signal dilaté dans le temps et pour la reproduction de l'électrocardiogramme d'un patient ;

e) un circuit de commande (12) qui, pendant la durée de l'affichage du signal dilaté dans le temps, interrompt temporairement la reproduction de l'électrocardiogramme du patient sur l'appareil d'affichage.

2. Dispositif selon la revendication 1, dans lequel on utilise comme appareil d'affichage un appareil d'enregistrement dans lequel la vitesse du papier est égale à environ 2,5 cm/s, caractérisé en ce que le facteur de dilatation dans le temps est égal environ à 100.

3. Dispositif selon la revendication 1, caractérisé en ce qu'il est prévu un écran pour l'affichage et qu'une mémoire intermédiaire est associée à cet écran.

4. Dispositif selon l'une des revendications 1 à 3, caractérisé en ce que la mémoire (4) est réalisée sous la forme d'une mémoire analogique pour au moins 100 mots possédant une longueur de 6 bits.

5. Dispositif selon l'une des revendications 1 à 4, caractérisé en ce qu'à la mémoire (4) se trouve associé, en vue de la commutation de la vitesse d'enregistrement à la vitesse de lecture, un générateur de cadence commutable (13), dont l'entrée de commande (14) est raccordée par l'intermédiaire d'une minuterie (16), qui est réglée sur le retard de réponse du défibrillateur ainsi que sur la durée maximum du courant traversant le patient, à un générateur d'impulsions de commande déclenchant la défibrillation.

6. Dispositif selon la revendication 5, dans lequel la durée de commutation du relais, qui déclenche l'impulsion du défibrillateur, dépend de la tension, caractérisé en ce que la minuterie (16), qui est branchée entre le générateur d'impulsions de commande et l'entrée de commande (14) du générateur de cadence (13), présente une durée de retard dépendant de la tension d'alimentation du relais.

7. Dispositif selon la revendication 5 ou 6, caractérisé en ce qu'à la sortie de la minuterie (16) est raccordé un circuit de retardement (15) déterminant la durée de l'interruption et dont le signal d'entrée est envoyé au circuit de commande (12) qui commute l'envoi du signal à l'appareil d'affichage entre une ligne (10) véhiculant le signal de l'électrocardiogramme et la sortie (7) de la mémoire (4).

8. Dispositif selon l'une des revendications 1 à 7, caractérisé en ce que le dispositif de découplage comporte un transformateur d'intensité branché dans le circuit défibrillateur/patient.

9. Dispositif selon l'une des revendications 1 à 7, comportant une bobine d'arrêt située dans le circuit défibrillateur/patient, caractérisé en ce que le dispositif de découplage comporte un enroulement (1) accouplé à la bobine d'arrêt, ainsi qu'un intégrateur (2) branché en aval.

FIG. 1

Integrator

Ausgangs-verstärker

Speicher

Taktgeber

Steuer-schaltung

Zeitgeber

Verzögerungs-schaltung

+12V

A

FIG. 2

2